# EUROPEAN PATENT APPLICATION

(11) **EP 4 353 785 A1**
(43) Date of publication of application: **17.04.2024**
(21) Application number: 22804677.7
(22) Date of filing: 17.05.2022
(51) Int. Cl.: C09C 1/00, C09C 1/28, C09C 3/06, A61Q 1/02, C09D 201/00, A61K 8/28, C09D 7/62

(54) **BRIGHT PIGMENT HAVING COATING FILM CONTAINING ZIRCONIUM OXIDE**

(30) Priority: 17.05.2021 JP 2021083520
(71) Applicant: NIPPON SHEET GLASS COMPANY, LIMITED, Tokyo 108-6321 (JP)
(72) Inventor: HORIGUCHI Haruko, Tokyo 108-6321 (JP); MIKAMI Shinji, Tokyo 108-6321 (JP)
(74) Representative: Cabinet Beau de Loménie
(86) International application number: PCT/JP2022/020518
(87) International publication number: WO 2022/244766

(57) **Abstract**

In light of diversification of required properties, the present disclosure seeks to improve glitter pigments from a viewpoint different from the intensity and vividness of color. The present disclosure provides a glitter pigment including: a flaky substrate; and a coating including zirconium oxide on the flaky substrate, wherein a mean deviation of mean friction coefficient (MMD) of the glitter pigment is 1.5 or less. The present disclosure also provides a glitter pigment including: a flaky substrate; and a coating including zirconium oxide on the flaky substrate, wherein a specific surface area of the glitter pigment is 0.5 cm²/g or more.

## Description

### TECHNICAL FIELD

The present invention relates to a glitter pigment, particularly relates to a glitter pigment including a coating on its surface.

### BACKGROUND ART

Glitter pigments are sometimes added to compositions, such as paints, inks, and cosmetics, whose appearances are seen as important. Glitter pigments with pearly luster are called pearlescent pigments, effect pigments, or the like. Glitter pigments generally include a flaky substrate made of glass, mica, or the like and a coating on the substrate. A coating including titanium oxide is commonly used as the coating. For more vivid colors, it has been proposed that fine metal particles typified by fine gold particles be adhered to a surface of a flaky substrate or a metal oxide film (for example, in Patent Literature 1).

### CITATION LIST

### Patent Literature

Patent Literature 1: JP 2006-299051 A

### SUMMARY OF INVENTION

### Technical Problem

Glitter pigments have been developed with emphasis on intense glitter and color vividness. However, with the increase of the variety of applications, properties required of glitter pigments are becoming diverse. The present invention aims to improve glitter pigments from a viewpoint different from the intensity and vividness of color.

### Solution to Problem

In one aspect, the present invention provides a glitter pigment including:
a flaky substrate; and
a coating including zirconium oxide on the flaky substrate, wherein
a mean deviation of mean friction coefficient (MMD) of the glitter pigment is 1.5 or less.

In another aspect, the present invention provides a glitter pigment including:
a flaky substrate; and
a coating including zirconium oxide on the flaky substrate, wherein
a specific surface area of the glitter pigment is 0.5 cm²/g or more.

In still another aspect, the present invention provides a pigment-including composition including the glitter pigment according to the present invention.
In yet another aspect, the present invention provides a painted article including:
a substrate; and
a coating film on the substrate, wherein
the coating film includes the glitter pigment according to the present invention.

In another aspect, the present invention provides a method for producing a glitter pigment, the glitter pigment including a flaky substrate and a coating including zirconium oxide on the flaky substrate, the method including:
bringing a liquid containing a tin-including salt into contact with a surface of a flaky substrate; and
forming a coating including zirconium oxide by liquid-phase deposition on the surface that was in contact with the liquid.

### Advantageous Effects of Invention

The glitter pigment according to the present invention can have features different from those of conventional glitter pigments. The features are, for example, smoothness on skin and a color, such as silver, a color between yellow and orange, red, or another color, that is not too intense.

### BRIEF DESCRIPTION OF DRAWINGS

FIG. 1 shows a result of scanning electron microscope (SEM) observation of a cross-section of an example of a glitter pigment.
FIG. 2 shows a result of SEM observation of a surface of an example of a glitter pigment.

### DESCRIPTION OF EMBODIMENTS

Hereinafter, embodiments of the present invention will be described. The following description is not intended to limit the present invention to specific embodiments.

The term "main component" herein refers to a component whose content is highest. An MMD is an abbreviation of a mean deviation of mean friction coefficient (MIU). The MMD is a measure of smoothness of skin texture. The lower the MMD is, the better the smoothness is. The MMD measurement method will be described in details in EXAMPLES.

The glitter pigment according to the present embodiment includes a flaky substrate and a coating including zirconium oxide (zirconium-oxide-including film) on the flaky substrate. The glitter pigment has at least one, preferably both, of the following features.
1) The MMD of the glitter pigment is 1.5 or less.
2) The specific surface area of the glitter pigment is 0.5 cm²/g or more.

Titanium oxide is known as a material that is used for formation of coatings and that can achieve a vivid color taking advantage of its high refractive index. However, there is room for consideration of an alternative material at least in terms of improvement of the skin texture. Through studies of various materials, the present inventor has found that zirconium oxide is suitable for achieving a low MMD. A preferred MMD of the glitter pigment is 1.0 or less, 0.8 or less, even 0.6 or less, or particularly 0.5 or less. The lower limit of the MMD is, for example, but not particularly limited to, 0.01 or more.

The type of the flaky substrate also affects the MMD. For example, it is generally difficult to achieve a low MMD in the case where a flaky substrate is mica, synthetic mica, alumina flakes, sericite, titania flakes, or iron oxide flakes. According to the present embodiment, however, an MMD of 1.5 or less, e.g., more than 1.0 and 1.5 or less can be achieved using any of these flaky substrates. According to the present embodiment, in the case where the flaky substrate is glass flakes or silica flakes, an MMD of 1.0 or less, or even a lower value (for example, 0.6 or less) within the above range can be achieved.

The glitter pigment according to the present embodiment can have an MIU of for example, 2.1 or less, preferably 2.0 or less, more preferably 1.5 or less, or, in some cases, 1.0 or less. The lower limit of the MIU is, for example, but not limited to, 0.01 or more.

Formation of the coating on the flaky substrate is commonly performed by liquid-phase deposition. To obtain the zirconium-oxide-including film having a sufficiently low MMD, a pretreatment is desirably performed for a surface on which zirconium oxide is to be deposited. An example of the pretreatment is a contact treatment of the surface using a liquid containing a tin-including salt.

It has been found out that the pretreatment using the liquid containing a tin-including salt increases the specific surface area of the glitter pigment. The increase in specific surface area can be a factor that contributes to improvement of the texture and other properties affected by the specific surface area. A preferred range of the specific surface area is 0.8 cm²/g or more, 1.0 cm²/g or more, 2.0 cm²/g or more, 2.5 cm²/g or more, 3.0 cm²/g or more, 3.5 cm²/g or more, or 4.0 cm²/g or more. The specific surface area may be 4.5 cm²/g or more. The upper limit of the specific surface area is, for example, but not particularly limited to, 100.0 cm²/g or less, 50.0 cm²/g or less, 20.0 cm²/g or less, even 18.0 cm²/g or less, or, in some cases, 15.0 cm²/g or less.

It has also been found out that although the glitter imparted by the zirconium-oxide-including film is relatively mild, such a glitter can rather be a factor for a high transparency in makeup cosmetics such as foundations. For the glitter pigment according to the present embodiment, in terms of an L*a*b* color system, L* of a reflected color measured using an illuminant D65 can be 30 or more and less than 70, even 35 or more and 68 or less, or particularly 40 or more and 65 or less. The L* value of a titanium-oxide-including film is commonly over 70. Moreover, by appropriate adjustment of the film thickness, the zirconium-oxide-including film can achieve colors desirable for makeup cosmetics, specifically, colors from silver to yellow, to orange, to red, and even to purple. The reflected color measurement method will be described in

### EXAMPLES.

The zirconium-oxide-including film is inferior to a titanium-oxide-including film in imparting intense glitter and an intense color. However, the zirconium-oxide-including film is superior to a titanium-oxide-including film in prevention of a complementary color, which is sometimes observed and salient on a titanium-oxide-including film. Salient appearing of a complementary color can result in observation of a slightly dull color produced by a glitter pigment. A complementary color tends to cause a problem when the amount of a glitter pigment added is large.

Hereinafter, the flaky substrate and the coating included in the glitter pigment will be described.

### (Flaky substrate)

The flaky substrate is a very small plate-shaped flake that is called, for example, a scaly substrate. The flaky substrate is, for example, at least one selected from the group consisting of a glass flake, an alumina flake, a graphite flake, an iron oxide flake, a titania flake, a silica flake, bismuth oxychloride, mica, talc, and sericite. The flaky substrate is preferably a glass flake, an alumina flake, or mica. The mica may be natural mica or synthetic mica. The glass flake, which is a particularly preferred flaky substrate, will be described hereinafter.

A glass composition forming the glass flake may be one including silicon oxide and a metal oxide other than silicon oxide, specifically, one including silicon oxide and at least one selected from the group consisting of aluminum oxide, calcium oxide, and sodium oxide. Silicon oxide may be the main component in the glass composition. The silicon oxide content in the glass composition is, for example, but not particularly limited to, 40 mass% or more, or 45 mass% or more, and may be 75 mass% or less. The glass composition may have a transmittance of 60%, or even more than 70% in the wavelength range of 400 to 800 nm, as calculated for a thickness of 100 µm. The glass composition may be, specifically, one that falls under at least one selected from the group consisting of soda-lime glass, A-glass, C-glass, E-glass, borosilicate glass, and aluminosilicate glass. The glass flake made of any of these multicomponent glass compositions is suitable for being produced by melting, which is superior in mass productivity.

A preferred average particle diameter of the glass flakes is 1 to 180 µm, 3 to 120 µm, or, in some cases, 5 to 100 µm. The average particle diameter of the glass flakes is determined as a particle diameter (D50) at 50% by volume in a cumulative undersize distribution of light scattering particle sizes measured by laser diffractometry. A preferred thickness of the glass flake is 0.1 to 10 µm, even 0.2 to 5 µm, or particularly 0.25 to 2 µm. Too large an average particle diameter and too large a thickness make it difficult to obtain a glitter pigment having a sufficiently good texture. However, as to the thickness, a very good texture can sometimes be obtained when the glass flake has a thickness of 1 to 3 µm. It should be noted that the above numerical ranges are also preferred average particle diameters and preferred thicknesses of the flaky substrate other than the glass flake.

The glass flakes can be produced, for example, by a blowing process. The blowing process includes: melting glass cullet; discharging the molten glass continuously through a circular slit while a gas such as air is blown into the molten glass from a blowing nozzle provided inwardly of the circular slit to inflate the molten glass into a balloon; and crushing the inflated, thinned glass into flakes (scaly particles). For example, commercially-available glass flakes on sale under the name of the "GLASFLAKE" (registered trademark) series by Nippon Sheet Glass Co., Ltd. can be used as the glass flakes.

The glass flake has a smoother and flatter surface than a crystalline particulate such as mica. Because of this, the glass flake is particularly suitable for achieving a sufficiently low MMD.

In a preferred embodiment, the glitter pigment includes the glass flake and a single-layer zirconium-oxide-including film formed directly on a surface of the glass flake. Alternatively, an underlayer film may be formed on a surface of the flaky substrate. In this case, the zirconium-oxide-including coating is formed on a surface of the underlayer film. The underlayer film may be a single-layer film or a multilayer film. The underlayer film may be a film including an oxide.

### (Zirconium-oxide-including film)

The zirconium-oxide-including film includes at least zirconium oxide. The zirconium-oxide-including film may include zirconium oxide as its main component. A preferred film thickness of the zirconium-oxide-including film is 40 to 160 nm, even 50 to 140 nm, particularly 70 to 120 nm, or, in some cases, 90 to 110 nm. A color produced by the zirconium-oxide-including film can be appropriately adjusted by controlling the film thickness. The color commonly shifts from silver to yellow to orange to red to purple as the film thickness increases. In a preferred embodiment, the surface of the zirconium-oxide-including film is bare.

In view of inclusion in makeup cosmetics, the film thickness of the zirconium-oxide-including film is preferably 50 to 140 nm so that colors from silver to red via yellow and orange are likely to be achieved. In view of inclusion in foundations, the film thickness of the zirconium-oxide-including film is preferably 50 to 120 nm so that colors from silver to orange via yellow are likely to be achieved. However, in the case of makeup cosmetics other than foundations, colors between red and purple, which are likely to be achieved at a film thickness in the range of more than 120 nm to 160 nm or less, can be useful.

Liquid-phase deposition is suitable for formation of the zirconium-oxide-including film on the flaky substrate. This liquid-phase deposition is commonly performed by dispersing the flaky substrate in a liquid containing a zirconium-including salt and then stirring the liquid. The liquid may be an aqueous solution in which the zirconium-including salt is dissolved. The zirconium-including salt is, for example, zirconium sulfate, zirconium oxychloride, or ammonium zirconium carbonate. If necessary, a pH adjuster such as an acid may be added into the liquid. The pH of the liquid can be adjusted, for example, in the range of 1.9 to 4.0, or even 2.0 to 3.5.

As described above, the pretreatment using the liquid containing a tin-including salt makes it easy to achieve a sufficiently low MMD of the zirconium-oxide-including film. How the MMD is decreased by this pretreatment is currently unclear; the pretreatment may affect the surface or the density of zirconium oxide generated thereby, thus decreasing the MMD. The liquid containing a tin-including salt may be an aqueous solution in which the tin-including salt is dissolved. The tin-including salt is, for example, tin chloride, more specifically, tin(II) chloride, tin(IV) chloride, tin(II) chloride dihydrate, or tin(IV) chloride pentahydrate.

Hereinafter, a composition including the glitter pigment and a painted article including the glitter pigment will be described.

### (Composition and painted article)

The pigment-including composition according to the present embodiment includes the glitter pigment according to the present embodiment. The composition is, for example, at least one selected from the group consisting of a paint, an ink, a cosmetic, and a resin composition. The resin composition is, for example, a molded artificial marble. The painted article according to the present embodiment includes a substrate and a coating film thereon, and the coating film includes the glitter pigment according to the present embodiment. The painted article is, for example, painted paper. However, the substrate is not limited to paper, and may be a metal, a resin, a ceramic, or the like. The coating film may include the pigment-including composition according to the present invention.

The features of the glitter pigment according to the present embodiment are often recognized as advantages in the case where the pigment is included in cosmetics. The cosmetics are not limited to particular ones, and may be basic skin care products such as creams, hair care cosmetics such as hair styling agents, body care products such as body creams, body powders, antiperspirants, and sunscreens, and bath additives. The cosmetics are preferably makeup cosmetics such as foundations, mascaras, eyeshadows, eyeliners, lipsticks, lip glosses, blushes, and manicures. The makeup cosmetics may be point makeup cosmetics such as lipsticks and blushes, or may be base makeup cosmetics such as concealers, foundations, and finishing powders.

The forms of the cosmetic is, for example, but not particularly limited to, a powder, a stick, a pencil, a cream, an emulsion, a dispersion, an oil, a tablet, a capsule, a liner, a paint, a gel, or the like. For example, the foundation may be any of a cake type, a powder type, a cream type, or a liquid type. The cosmetic can include a conventional component as a component other than the glitter pigment. The component other than the glitter pigment is selected as appropriate according to the cosmetic type. The cosmetic may include a pigment other than the glitter pigment.

For example, a body pigment, a colorant, a white pigment, a pearlescent or effect pigment, and the like are incorporated in the foundation. The body pigment is included to impart spreadability, adherability, and even luster, and also serves as a bulking agent. The colorant is included to impart a basic color tone. The white pigment is included to impart a hiding power and whiteness. The pearlescent pigment is included to impart a pearly luster (pearl effect). The amount of the pearlescent pigment incorporated in the foundation for partial luster is at most about 8 mass% of the total amount of the foundation. On the other hand, the body pigment is a basic component that forms, so to speak, a frame of the foundation. The amount of the body pigment incorporated in the foundation is commonly larger than the above amount so as to adjust the properties of the whole of the foundation.

The cosmetic may include a small amount of the glitter pigment according to the present embodiment, in other words, such a small amount that the glitter pigment serves as a pearlescent pigment. The cosmetic may include a relatively large amount of the glitter pigment according to the present embodiment, in other words, such a large amount that the glitter pigment serves substantially as a body pigment or a similar pigment. In the latter case, the amount of the glitter pigment may be, for example, 10 mass% or more, or particularly 12 to 30 mass% of the total amount of the cosmetic.

Specific examples of the present invention will be hereinafter described by examples. The examples given below are not intended to limit the present invention. The properties of glitter pigments were evaluated in the following manner.

### (Texture: MIU and MMD)

The MIU representing the slipperiness and the MMD representing the smoothness were measured using a friction tester "KES-SE" manufactured by KATO TECH CO., LTD. A10 mm² silicone device was used as a friction contactor, and the measurement conditions were 50 g of a static load and a measurement speed of 1 mm/sec. A specimen to be measured was produced by uniformly spraying a glitter pigment to an artificial black leather ("Protein Leather (registered trademark) Saprare" manufactured by IDEATEX JAPAN Co., Ltd.) so that the mass per unit area would be 1.3 mg/cm². Incidentally, the relative humidity was 54% during the measurement.

### (Reflected light: lightness and color)

The lightness L* of reflected light was measured using a chromometer "CR-400" manufactured by KONICA MINOLTA, INC. Each glitter pigment was added to and mixed with a transparent acrylic resin (Nippe Acryl "Auto Clear Super" manufactured by NIPPON PAINT Co., Ltd.) so that the glitter pigment concentration would be 10 mass%, and the resulting composition was applied onto a white-black hiding-chart to produce a specimen to be measured. The composition was applied such that the coating film had a thickness of 9 mil (about 228.6 µm), but the coating film had a thickness of 70 to 80 µm after dried. A light source used was an illuminant D65, and a measured surface was the coated black surface. The reflected light measured was vertical reflected light. The reflected light measured was observed using the CIE 2° field of view color matching functions.

### (Film thickness, substrate thickness)

A cross-section of each glitter pigment was observed using a scanning electron microscope (SEM) for measurement of the film thickness of the coating and the thickness of the substrate.

### (Average particle diameter D50)

Each flaky substrate to be measured was dispersed in water and measured for the average particle diameter thereof, namely, the particle diameter (D50) at a cumulative volume percentage of 50%, using "MT3300" manufactured by Microtrac.

### (Specific surface area)

A BET specific surface area was measured by a nitrogen gas method using "NOVA 4200e" manufactured by Quantachrome Instruments, Inc.

### (Specimen 1)

### •Pretreatment of flaky substrate

Glass flakes having an average particle diameter (D50) of 80 µm and a thickness of 1.3 µm were used as a flaky substrate. All of the glass flakes used in this example and the examples below are GLASFLAKE (registered trademark) products manufactured by Nippon Sheet Glass Co., Ltd., and are each made of a multicomponent glass. Each multicomponent glass includes SiO₂, Al₂O₃, MgO, and CaO and further includes an alkaline component (at least one selected from the group consisting of Li₂O, Na₂O, and K₂O). First, 150 g of the flaky substrate was mixed with 1.5 L of water, and the mixture was stirred. Next, hydrochloric acid was added thereto to adjust the pH within 1.45 to 1.55. Subsequently, 9.06 mL of a 1.3 mass% aqueous tin(IV) chloride solution was added thereto. The stirring was further continued, and powder and liquid were separated by filtration. The separated powder was dispersed in water, and the dispersion was filtered again to obtain a pretreated flaky substrate.

### •Formation of zirconium-oxide-including film

An amount of 150 g of the pretreated flaky substrate and 1.5 L of water were mixed, and the temperature was increased to 75±3°C. Next, hydrochloric acid and sodium hydroxide were added thereto to adjust the pH within 2.0 to 3.5. Subsequently, without changing the above pH, a 20 mass% aqueous zirconium sulfate solution and a 10 mass% aqueous sodium hydroxide solution were simultaneously dropped for 4 hours to coat the zirconium oxide film. After that, powder and liquid were separated by filtration. A step in which the separated powder was dispersed in an aqueous solution having a pH adjusted within 2.0 to 3.5 with hydrochloric acid and the dispersion was stirred and then filtered was performed twice, and, subsequently, a step in which the powder was dispersed in water and the dispersion was stirred and then filtered was performed twice. Finally, the resulting powder was dried in an atmosphere at 180°C for 12 hours and was then sintered at 600°C for 2 hours. A flaky substrate with a zirconium-oxide-including film on its surface was obtained in this manner.

### (Specimen 2)

A flaky substrate with a zirconium-oxide-including film on its surface was obtained in the same manner as in Specimen 1, except that glass flakes having an average particle diameter (D50) of 25 µm and a thickness of 0.5 µm were used as the flaky substrate.

### (Specimen 3)

A flaky substrate with a zirconium-oxide-including film on its surface was obtained in the same manner as in Specimen 1, except that glass flakes having an average particle diameter (D50) of 8 µm and a thickness of 0.3 µm were used as the flaky substrate.

### (Specimen 4)

A flaky substrate with a zirconium-oxide-including film on its surface was obtained in the same manner as in Specimen 1, except that the dropping time during which the aqueous zirconium sulfate solution and the aqueous sodium hydroxide solution were dropped was 3 hours 30 minutes in the step of forming the zirconium-oxide-including film.

### (Specimen 5)

A flaky substrate with a zirconium-oxide-including film on its surface was obtained in the same manner as in Specimen 4, except that glass flakes having an average particle diameter (D50) of 18 µm and a thickness of 1.3 µm were used as the flaky substrate.

### (Specimen 6)

A flaky substrate with a zirconium-oxide-including film on its surface was obtained in the same manner as in Specimen 4, except that glass flakes having an average particle diameter (D50) of 8 µm and a thickness of 0.3 µm were used as the flaky substrate.

### (Specimen 7)

A flaky substrate with a zirconium-oxide-including film on its surface was obtained in the same manner as in Specimen 4, except that glass flakes having an average particle diameter (D50) of 120 µm and a thickness of 1.3 µm were used as the flaky substrate.

### (Specimen 8)

A flaky substrate with a zirconium-oxide-including film on its surface was obtained in the same manner as in Specimen 4, except that glass flakes having an average particle diameter (D50) of 160 µm and a thickness of 1.3 µm were used as the flaky substrate.

### (Specimen 9)

A flaky substrate with a zirconium-oxide-including film on its surface was obtained in the same manner as in Specimen 1, except that the dropping time was 4 hours 30 minutes in the step of forming the zirconium-oxide-including film.

### (Specimen 10)

A flaky substrate with a zirconium-oxide-including film on its surface was obtained in the same manner as in Specimen 1, except that the dropping time was 4 hours 45 minutes in the step of forming the zirconium-oxide-including film.

### (Specimen 11)

A flaky substrate with a zirconium-oxide-including film on its surface was obtained in the same manner as in Specimen 1, except that the dropping time was 6 hours in the step of forming the zirconium-oxide-including film.

### (Specimen 12)

A zirconium-oxide-including film was formed in the following manner without the pretreatment of a flaky substrate. The flaky substrate used is the same as that in Specimen 1.

### •Formation of zirconium-oxide-including film

Hydrochloric acid was added to 150 mL of water to adjust the pH within 2.0 to 3.5. An amount of 0.5 g of urea and 21 g of zirconium sulfate were sequentially added thereto. An amount of 6 g of the flaky substrate was added thereto, and the mixture was stirred further. After that, powder and liquid were separated by filtration. The obtained powder was washed with water, dried at 90°C for 1.5 hours, and then sintered at 800°C for 1 hour. A flaky substrate with a zirconium-oxide-including film on its surface was obtained in this manner.

### (Specimen 13)

"METASHINE (registered trademark) MT1080RS" manufactured by Nippon Sheet Glass Co., Ltd. was used.

### (Specimen 14)

A flaky substrate with a zirconium-oxide-including film on its surface was obtained in the same manner as in Specimen 1, except that a synthetic mica manufactured by NIHON KOKEN KOGYO CO., LTD. and having an average particle diameter (D50) of 10 µm was used as the flaky substrate. The synthetic mica had a thickness of several micrometers or less.

### (Specimen 15)

"Timiron Ice Crystal" manufactured by Merck KGaA and including a synthetic mica as a flaky substrate and a titanium-oxide-including film on a surface of the synthetic mica was used.

Tables 1 and 2 show the evaluation results. It should be noted that "-" in Tables 1 and 2 indicates no measurement and that "*" in Table 2 indicates "a wide range from one submicron to several micrometers".

**[Table 1]**

| Specimen | Substrate (µm) | | | Film (nm) | | Reflected light | | Texture | Specific surface area (cm²/g) |
|---|---|---|---|---|---|---|---|---|---|
| | Material | D50 | Thickness | Main component | Thickness | Color | L^{*} | MMD | |
| 1 | Glass | 80 | 1.3 | ZrO₂ | 90 | Silver | 54.4 | 0.33 | 5.384 |
| 2 | Glass | 25 | 0.5 | ZrO₂ | 90 | Silver | 59.8 | 0.36 | 15.110 |
| 3 | Glass | 8 | 0.3 | ZrO₂ | 90 | Silver | 55.2 | 0.34 | 15.839 |
| 4 | Glass | 80 | 1.3 | ZrO₂ | 80 | Silver | - | 0.30 | 4.500 |
| 5 | Glass | 18 | 1.3 | ZrO₂ | 80 | Silver | - | 0.26 | 5.805 |
| 6 | Glass | 8 | 0.3 | ZrO₂ | 80 | Silver | - | 0.30 | 15.243 |
| 7 | Glass | 120 | 1.3 | ZrO₂ | 80 | Silver | - | 0.34 | 4.832 |
| 8 | Glass | 160 | 1.3 | ZrO₂ | 80 | Silver | - | 0.54 | 4.321 |
| 9 | Glass | 80 | 1.3 | ZrO₂ | 110 | Yellow to orange | - | 0.35 | 5.285 |
| 10 | Glass | 80 | 1.3 | ZrO₂ | 130 | Red | - | 0.31 | 5.231 |
| 11 | Glass | 80 | 1.3 | ZrO₂ | 160 | Purple | - | 0.33 | 4.920 |
| 12 | Glass | 80 | 1.3 | ZrO₂ | 90 | Silver | 28.6 | 0.77 | 0.188 |
| 13 | Glass | 80 | 1.3 | TiO₂ | 60 | Silver | 73.0 | 1.31 | 2.139 |

| | | | | | | | | | |
|---|---|---|---|---|---|---|---|---|---|
| •The tin pretreatment was not performed in Specimen 12. | | | | | | | | | |

**[Table 2]**

| | Substrate (unit: µm) | | | Film (unit: nm) | | Reflected light | | Texture |
|---|---|---|---|---|---|---|---|---|
| | Material | D50 | Thickness | Main component | Thickness | Color | L^{*} | MMD |
| 14 | Mica | 10 | * | Zirconium oxide | 90 | Silver | 47.3 | 1.48 |
| 15 | Mica | 100 | * | Titanium oxide | - | Silver | 70.7 | 1.55 |

| | | | | | | | | |
|---|---|---|---|---|---|---|---|---|
| •The thicknesses of the micas distribute in a wide range from one submicron to several micrometers. | | | | | | | | |

According to Tables 1 and 2, the zirconium-oxide-including films are superior to the titanium-oxide-including films in terms of smoothness imparted by the glitter pigments. The smoothness is remarkably improved when glass flakes are used as the flaky substrate. Tables 1 and 2 also indicates that the zirconium-oxide-including films are more suitable for achieving reduced lightness than the titanium-oxide-including films. Tables 1 and 2 also indicates that the tin pretreatment of the flaky substrate contributes to a decrease in MMD and an increase in specific surface area.

The MIUs of Specimens 1 to 13 are within 0.8 to 3.8. The MIU is 1.5 or less for Specimens 1, 4, and 9 to 11 each including: the flaky substrate having a D50 of 100 µm or less and a thickness of 1 to 3 µm; and the zirconium-oxide-including film.

Foundations were prepared using the glitter pigments of Specimens 4 to 11, 13, and 15 according to the formulation shown in Table 3. Each foundation was applied to skin, and the appearance was evaluated according to the following criteria.
4 points: The skin looks natural and bright. The skin does not glare, either.
3 points: The skin looks bright but glares a little.
2 points: The color of the skin is slightly unnatural.
1 point: The skin unnaturally glares.

**[Table 3]**

| (Foundation formulation) | | | |
|---|---|---|---|
| | Component | Supplier and product name | Mass% |
| body pigment | Talc | ASADA MILLING CO., LTD., JA46-R | 35.34 |
| | Sericite | Sanshin Mining Ind. Co., Ltd., sericite FSE | 15.00 |
| | Glitter pigment | Refer to Tables 1 and 2 | 15.00 |
| White pigment | Titanium oxide | Tayca Corporation, MP-1133 | 8.00 |
| colorant | Yellow iron oxide | DIC, C33-9001 | 3.10 |
| | Red iron oxide | DIC, C33-8001 | 0.86 |
| | Black iron oxide | DIC, C33-7001 | 0.28 |
| Spherical powder | Silica, titanium oxide | NSG, i-NAFLECS (registered trademark) IWS22S13 | 7.00 |
| | Titanium oxide (Al, stearic acid treatment) | Tayca Corporation, MT-100TV | 5.00 |
| Oil | Methylphenyl polysiloxane | Shin-Etsu Silicones, KF-56 | 1.00 |
| | Methylpolysiloxane | Shin-Etsu Silicones, KF-96A-100cs | 1.00 |
| | Isostearyl alcohol | The Nisshin OilliO Group, Ltd., COSMOL 222 | 2.00 |
| | Triethylhexanoin | NIPPON FINE CHEMICAL CO., LTD., T.I.O | 2.00 |
| Surfactant | Sorbitan sesquiisostearate | The Nisshin OilliO Group, Ltd., COSMOL 182V | 1.00 |
| Ultraviolet absorber | Octyl methoxycinnamate, tocopherol | The Nisshin OilliO Group, Ltd., NOMUCOAT TAB | 3.00 |
| Antioxidant | Natural vitamin E | Eisai Food & Chemical Co., Ltd., E-MIX D | 0.02 |
| Antiseptic agent | Ethylparaben | Ueno Fine Chemicals Industry, Ltd. | 0.40 |
| Total | | | 100.00 |

Table 4 shows the results along with the textures (MIUs and MMDs) of the glitter pigments.

**[Table 4]**

| Specimen | Substrate (unit: µm) | | Film (unit: nm) | | Reflected light | Texture | | Appearance of foundation (point) |
|---|---|---|---|---|---|---|---|---|
| | D50 | Thickness | Main component | Thickness | Color | MIU | MMD | |
| 4 | 80 | 1.3 | Zirconium oxide | 80 | Silver | 0.83 | 0.30 | 4 |
| 5 | 18 | 1.3 | Zirconium oxide | 80 | Silver | 1.7 | 0.26 | 4 |
| 6 | 8 | 0.3 | Zirconium oxide | 80 | Silver | 1.8 | 0.30 | 4 |
| 7 | 120 | 1.3 | Zirconium oxide | 80 | Silver | 2.1 | 0.34 | 4 |
| 8 | 160 | 1.3 | Zirconium oxide | 80 | Silver | 2.1 | 0.54 | 3 |
| 9 | 80 | 1.3 | Zirconium oxide | 110 | Yellow to orange | 0.94 | 0.35 | 4 |
| 10 | 80 | 1.3 | Zirconium oxide | 130 | Red | 0.90 | 0.31 | 2 |
| 11 | 80 | 1.3 | Zirconium oxide | 160 | Purple | 0.83 | 0.33 | 2 |

The glitter pigments (Specimens 13 and 15) including the titanium-oxide-including films were incorporated in foundations according to the same formulation as above and were evaluated for their appearances; the results were both 1 point. The glitter pigments including the zirconium-oxide-including films are superior as pigments configured to be added to adjust the properties of a whole foundation to the glitter pigment including the titanium-oxide-including films.

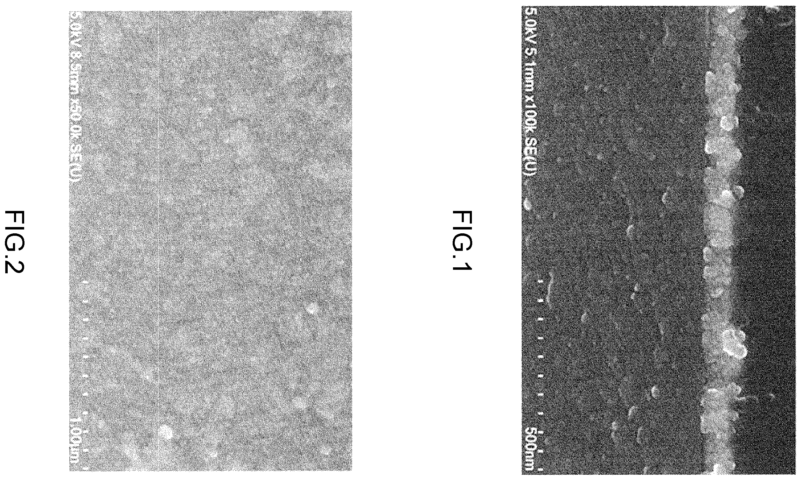

FIGS. 1 and 2 show the results of SEM observation of a flaky substrate with a zirconium-oxide-including film thereon. A cross-section in FIG. 1 shows the zirconium-oxide-including film having a film thickness of 90 nm. As shown in FIGS. 1 and 2, the zirconium-oxide-including film is closely packed with particles of about several tens of nanometers in size.

## Claims

1. A glitter pigment comprising:
a flaky substrate; and
a coating including zirconium oxide on the flaky substrate, wherein
a mean deviation of mean friction coefficient (MMD) of the glitter pigment is 1.5 or less.

2. A glitter pigment comprising:
a flaky substrate; and
a coating including zirconium oxide on the flaky substrate, wherein
a specific surface area of the glitter pigment is 0.5 cm²/g or more.

3. The glitter pigment according to claim 2, wherein a mean deviation of mean friction coefficient (MMD) of the glitter pigment is 1.5 or less.

4. The glitter pigment according to any one of claims 1 to 3, wherein the mean deviation of mean friction coefficient (MMD) is 0.6 or less.

5. The glitter pigment according to any one of claims 1 to 4, wherein a specific surface area of the glitter pigment is 2.5 cm²/g or more.

6. The glitter pigment according to any one of claims 1 to 5, wherein, in terms of an L*a*b* color system, L* of a reflected color is 30 or more and less than 70.

7. The glitter pigment according to any one of claims 1 to 6, wherein an average particle diameter of the flaky substrate is 180 µm or less.

8. The glitter pigment according to any one of claims 1 to 7, wherein the flaky substrate is a glass flake.

9. The glitter pigment according to any one of claims 1 to 8, wherein a film thickness of the coating is in a range of 40 nm to 160 nm.

10. The glitter pigment according to claim 9, wherein the film thickness of the coating is 120 nm or less.

11. A pigment-including composition comprising the glitter pigment according to any one of claims 1 to 10.

12. The pigment-including composition according to claim 11, being a makeup cosmetic.

13. A painted article comprising:
a substrate; and
a coating film on the substrate, wherein
the coating film includes the glitter pigment according to any one of claims 1 to 10.

14. A method for producing a glitter pigment, the glitter pigment including a flaky substrate and a coating including zirconium oxide on the flaky substrate, the method comprising:
bringing a liquid containing a tin-including salt into contact with a surface of a flaky substrate; and
forming a coating including zirconium oxide by liquid-phase deposition on the surface that was in contact with the liquid.
